# EUROPEAN PATENT APPLICATION

(11) **EP 0 858 813 A2**
(43) Date of publication of application: **19.08.1998**
(21) Application number: 98300939.0
(22) Date of filing: 10.02.1998
(51) Int. Cl.: A61M 37/00

(54) **Improvements in or relating to implanters**

(30) Priority: 15.02.1997 GB 9703166
(71) Applicant: Sterimatic Holdings Limited, Road Town Tortola British Virgin Islands (VG)
(72) Inventor: Rowley, Paul, Stroud, Gloucestershire, GL5 2JD (GB)
(74) Representative: Harding, Richard Patrick

(57) **Abstract**

An implanter 1 for introducing an implant 6 under the skin of a human or animal body comprises a needle holder 3 for a hollow needle 4 having a tip 5 within which the implant 6 is accommodated and by means of which the implant 6 is introduced under the skin, a plunger 7 for relative movement within the needle 4, and a depth guide in the form of a hollow sleeve 15 for contacting the surface of the skin during introduction of the needle tip 5 under the skin. The hollow sleeve 15 serves to effect relative movement between the needle 4 and the plunger 7 when the needle tip 5 has been introduced under the skin to a required depth in order to cause the implant 6 to be left at the required depth when the needle tip 5 is withdrawn. Such an implanter 1 allows the implant 6 to be located accurately within the body and thus avoids the problems associated with incorrect placing of the implant.

## Description

This invention relates to implanters for introducing implants into a human or animal body.

There are a number of applications in which it is required to introduce an implant under the skin of a human or animal. For example it is a common practice to provide a domestic animal with an identity tag in the form of an electronic implant which is introduced under the animal's skin and which can be subsequently detected by a scanner externally of the animal's body in order to ascertain the animal's identity and possibly to retrieve other data relating to the animal.

However a number of difficulties have been encountered in the introduction of such electronic implants into an animal's body. Because conventional implanters for introducing such implants into an animal's body simply comprise a needle having a hollow tip for accommodating the implant, and a plunger which can be operated to push the implant out beyond the needle tip into the animal's body when the needle tip has been introduced under the animal's skin, it is not possible to accurately control the position in which the implant is left in the animal's body. This can result in migration of the implant within the animal's body making it difficult to subsequently locate the implant and possibly causing medical problems as a result of such migration. Furthermore, since the implant is only loosely held within the needle tip, the implant can easily fall out of the needle tip and be lost prior to introduction into the animal's body. There is also a danger that the user will be accidentally pierced by the needle tip after it has been used to place the implant in the animal's body, and this can result in infection and transmission of blood borne diseases.

It is an object of the invention to provide an improved implanter for introducing implants into human or animal bodies.

According to the present invention there is provided an implanter for introducing an implant under the skin of a human or animal body, the implanter comprising a needle holder for a hollow needle having a tip within which the implant is accommodated and by means of which the implant is introduced under the skin, a plunger for relative movement within the needle, and a depth guide for contacting the surface of the skin during introduction of the needle tip under the skin and for effecting relative movement between the needle and the plunger when the tip of the needle has been introduced under the skin to a required depth in order to cause the implant to be left at the required depth when the needle tip is withdrawn.

Because the implant is not pushed out beyond the needle tip but instead is caused to be left behind at the required depth, such an implanter allows the implant to be located accurately within the body and thus avoids the problems associated with incorrect placing of the implant. Furthermore the implant can be held more positively within the needle tip in such an implanter, thus substantially removing the risk of the implant falling out of the needle tip and being lost before it has been introduced into the body. This is particularly advantageous in the case of electronic tag implants as such implants are costly and are small enough to be easily lost. The implanter also effectively removes the risk of the implanter being damaged during placement, or of additional components being left behind within the body.

In a preferred embodiment of the invention needle shielding means is provided for shielding the tip of the needle when it has been withdrawn from the skin. For example, the needle shielding means may be constituted by a part of the depth guide into which the tip of the needle is retractable after completion of implantation.

Furthermore it is preferred that locking means is provided to hold the needle in a retracted position in which the tip of the needle is shielded by the shielding means and accidental exposure of the tip of the needle is prevented.

In one embodiment the locking means comprises a latch which serves to couple together the shielding means and the needle holder in order to prevent relative movement between the needle and the shielding means in such a manner as to expose the tip of the needle.

In another embodiment the locking means comprises a resilient finger which is overridden by the tip of the needle when it is retracted into the needle shielding means and which prevents accidental exposure of the tip of the needle.

The plunger is advantageously provided with an extension which holds the implant within the tip of the needle until it is expelled by the plunger. For example, the extension may comprise a thin tailpiece which projects for the end of the plunger so as to hold the implant between the tailpiece and an inside wall of the needle.

Furthermore the implanter conveniently incorporates a substantially cylindrical handgrip from which the needle axially extends and which is adapted to be grasped by the user during introduction of the implant.

In one embodiment of the invention a spring is provided to effect automatic retraction of the needle relative to the plunger to expel the implant from the needle when the tip of the needle has been introduced under the skin to the required depth.

In this case the depth guide may be adapted to be axially displaced by contact with the skin in order to release a coupling between the needle and the plunger so as to permit retraction of the needle by the spring.

In order that the invention may be more fully understood, three implanters in accordance with the invention will now be described, by way of example, with reference to the accompanying drawing, in which:
Figure 1 is an axial section through a first implanter;
Figure 2 is a side view of a component part of the first implanter;
Figure 3 is an axial section through a second implanter, Figure 3A showing a detail of a possible variant;
Figure 4 is an axial section through a third implanter; and
Figure 5 is a cross-section taken along the line A-A in Figure 4.

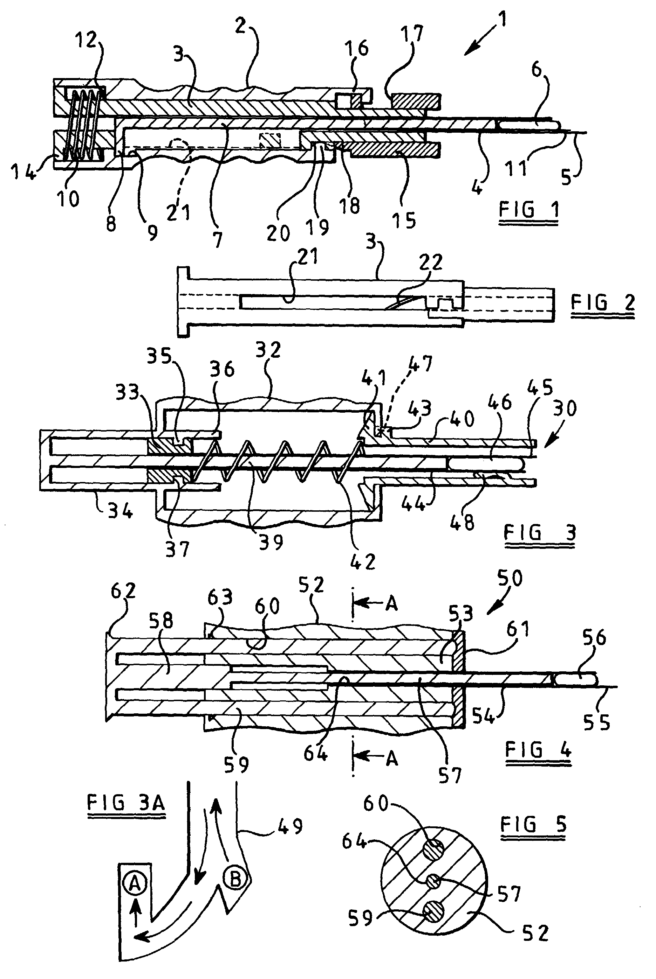

Figure 1 shows an implanter 1 comprising a generally cylindrical hollow handgrip 2 having an outer surface which is adapted to be grasped by the user's hand and accommodating therein a needle holder 3 which is also of generally cylindrical hollow form. The needle holder 3 has a hollow needle 4 received fixedly or detachably within one end, the needle 4 having a hollow tip 5 which is of sufficient internal diameter to accommodate the implant 6 therein. Furthermore an elongate plunger 7 extends axially within the needle holder 3 and within the needle 4 so as to contact the implant 6 within the needle tip 5, the plunger 7 having a transversely extending projection 8 at one end which is received with a slotted recess 9 in the inside wall of the handgrip 2. Furthermore a compression spring 10 is held under compression between an internal shoulder 12 of the handgrip 2 and an outer flange 14 on the needle holder 3. A depth guide in the form of a hollow sleeve 15 is held on the end of the needle holder 3 by an extension 16 on the end of the handgrip 2 which engages within a slot 17 in the wall of the sleeve 15.

In use of the implanter 1 to introduce the implant 6 under an animal's skin, the implanter 1 is held by its handgrip 2 after removal of a cap (not shown) from the needle tip 5, and the needle tip 5, with the implant 6 received therein, is caused to puncture the animal's skin. The needle tip 5 is then inserted to a depth of typically 30 mm until the end of the sleeve 15 contacts the surface of the animal's skin, and the sleeve 15 is pushed backwards relative to the handgrip 2 to the extent permitted by engagement of the extension 16 within the slot 17 and causes a projecting tab 18 on the sleeve 15 to engage a projection 19 on the inside wall of the handgrip 2 to cause disengagement of the projection 19 from a recess 20 in the needle holder 3. This releases the coupling between the handgrip 2 and the needle holder 3 and causes the needle holder 3 to be moved backwards by the spring 10 in relation to the handgrip 2, thus withdrawing the needle tip 5 from the animal and leaving the implant 6 in the required position under the animal's skin. The plunger 7 remains fixed relative to the handgrip 2 and therefore assists in expelling the implant 6 from the needle tip 5.

As the needle 4 is withdrawn from the animal's skin, the needle holder 3 is drawn back further relative to the handgrip 2 and the plunger 7 which is engaged with the handgrip 2 by the projection 8 extending through a slot 21 in the needle holder 3, as best seen in the side view of the needle holder 3 alone as shown in Figure 2. The needle holder 3 is drawn back until the projection 8 is overridden by a flexible finger 22 within the slot 21. The finger 22 prevents subsequent movement of the projection 8 back along the slot 21 and thus locks the needle holder 3 within the handgrip 2 in a position such that the needle tip 5 is shielded by the sleeve 15 and it is not possible for the user to be injured by accidentally being stuck by the needle 4.

Figure 3 shows an alternative implanter 30 having a handgrip 32 and a needle holder 33 held within a cylindrical portion 34 of the handgrip 32 by means of projections 35 on two diametrically opposite fingers 36 engaging within a circular groove 37 in the outer surface of the needle holder 33. The needle 44 connected to the needle holder 33 has a hollow needle tip 45 for accommodating the implant 46 and the plunger 39 is integrally formed with the handgrip portion 34 and extends along the needle 44. Furthermore a depth guide in the form of a sleeve 40 is coupled to the handgrip 32 by a circular flange 41 which is accommodated within the handgrip 32, a compression spring 42 acting between the needle holder 33 and the flange 41 on the sleeve 40.

In use of the implanter 30 to introduce the implant 46 under the animal's skin, the needle tip 45 is first exposed by twisting the sleeve 40 about its axis so as to align a tab 43 on the sleeve 40 with a corresponding recess as shown in broken lines at 47 in Figure 3. This unlocks the sleeve 40 from the handgrip 32 and permits the sleeve 40 to retract within the handgrip 32 whilst the needle tip 45 is introduced into the animal. In a variant of such an unlocking arrangement, a small projection could be provided at the outer circumference of the circular flange 41 which engages within a shaped track 49, as shown in Figure 3A, provided on the inside surface of the handgrip 32. When the projection is at a position A within the track 49, retraction of the sleeve 40 is prevented. However the sleeve may be manually twisted to place the projection in the position B within the track 49, and this then allows retraction of the sleeve 40 within the handgrip 32.

During introduction of the needle tip 45 into the animal, the sleeve 40 contacts the animal's skin and is forced backwardly so as to compress the spring 42 until the flange 41 on the sleeve 40 contacts the fingers 36 and pushes the fingers 36 apart so as to release the projections 35 from the groove 37 when the needle tip 45 has reached the correct depth. This releases the needle holder 33 and causes it to be forced backwardly by the spring 42 so as to retract the needle 44 leaving the implant 46 in position under the animal's skin. As the needle tip 45 is withdrawn from the animal it will come to rest in a position within the sleeve 40 in which the needle tip 45 is shielded by the sleeve 40 and a resilient finger 48 on the inside wall of the sleeve 40 extends in front of the needle tip 45 so as to prevent the needle tip 45 from being further exposed. The sleeve 40 may then be twisted to lock it in its original position, or alternatively the projection may be automatically caused to enter the locking position A in the variant of Figure 3A.

Figure 4 shows an implanter 50 comprising a handgrip 52 which is integrally formed with a needle holder 53 for holding the needle 54 having a tip 55 within which the implant 56 is received. In this case the plunger 57 is slidable relative to the handgrip 52 within a bore 64 and is integrally formed with a slide part 58 having two guide rods 59 extending through two guide bores 60 in the handgrip 52, as best seen in Figure 5. The guide rods 59 are in turn connected to a depth guide in the form of an end cap 61.

In use of the implanter 50 to introduce the implant 56 under the animal's skin, the needle 54 is first uncapped and the needle tip 55 is then introduced into the animal whilst exerting pressure on the slide part 58 in a direction towards the animal. When the needle tip 55 reaches the required depth as indicated by the end cap 61 contacting the surface of the skin, the user pulls the handgrip 52 in a direction away from the animal whilst still pushing the slide part 58 towards the animal so as to cause the needle tip 55 to be withdrawn leaving the implant 56 in position within the animal. When the handgrip 52 has been drawn back to a sufficient extent the needle tip 55 is shielded by the end cap 61 and is locked in position by engagement of ears 62 on the end of the slide part 58 within recesses 63 in the handgrip 52.

The above-described implanters 1, 30 and 50 all serve the intended function of placing the implant at the required depth under the animal's skin without any special measures having to be taken. Furthermore, in each of these embodiments, the plunger is provided with an extension in the form of a thin tailpiece 11, as best seen in Figure 1, which lies between the implant 6 and the inside wall of the needle 4 and which serves to jam the implant 6 within the needle tip 5 until expulsion of the implant 6 from the needle tip 5 is effected by relative movement of the needle 4 and the plunger 7. Whilst such an implanter is particularly suitable for placement of an electronic tag implant, it can also be used with other types of implant, such as a slow release pharmaceutical implant for the release of hormonal or therapeutic agents, for example.

## Claims

1. An implanter for introducing an implant under the skin of a human or animal body, the implanter (1, 30, 50) comprising a needle holder (3, 33, 53) for a hollow needle (4, 44, 54) having a tip (5, 45, 55) within which the implant (6, 46, 56) is accommodated and by means of which the implant is introduced under the skin, and a plunger (7, 39, 57) for relative movement within the needle, characterised in that the implanter (1, 30, 50) incorporates a depth guide (15, 40, 61) for contacting the surface of the skin during introduction of the needle tip under the skin and for effecting relative movement between the needle and the plunger when the tip of the needle has been introduced under the skin to a required depth in order to cause the implant to be left at the required depth when the needle tip is withdrawn.

2. An implanter according to claim 1, characterised in that needle shielding means (15, 40, 61) is provided for shielding the tip of the needle when it has been withdrawn from the skin.

3. An implanter according to claim 2, characterised in that the needle shielding means is constituted by a part of the depth guide (15, 40, 61) into which the tip of the needle is retractable after completion of implantation.

4. An implanter according to claim 2 or 3 characterised in that locking means (22, 48, 62, 63) is provided to hold the needle in a retracted position in which the tip of the needle is shielded by the needle shielding means (15, 40, 61) and accidental exposure of the tip of the needle is prevented.

5. An implanter according to claim 4, characterised in that the locking means comprises a latch (22, 62, 63) which serves to couple together the needle shielding means and the needle holder in order to prevent relative movement between the needle and the needle shielding means in such a manner as to expose the tip of the needle.

6. An implanter according to claim 4 or 5, characterised in that the locking means comprises a resilient finger (48) which is overridden by the tip of the needle when it is retracted into the needle shielding means and which prevents accidental exposure of the tip of the needle.

7. An implanter according to any preceding claim, characterised in that the plunger (7, 39, 57) is provided with an extension (11) which holds the implant within the tip of the needle until it is expelled by the plunger.

8. An implanter according to any preceding claim, characterised in that the implanter (1, 30, 50) incorporates a substantially cylindrical handgrip (2, 32, 52) from which the needle axially extends and which is adapted to be grasped by the user during introduction of the implant.

9. An implanter according to any preceding claim, characterised in that a spring (10, 42) is provided to effect automatic retraction of the needle relative to the plunger to expel the implant from the needle when the tip of the needle has been introduced under the skin to the required depth.

10. An implanter according to claim 9, characterised in that the depth guide (15, 40) is adapted to be axially displaced by contact with the skin in order to release a coupling between the needle (4, 44) and the plunger (7, 39) so as to permit retraction of the needle by the spring.
